Europäisches Patentamt

European Patent Office

Office européen des brevets

(19)

(11) Publication number: **0 400 463**
A·1

## EUROPEAN PATENT APPLICATION

(12)

(21) Application number: **90109749.3**

(22) Date of filing: **22.05.90**

(51) Int. Cl.⁵: **A61M 1/00**

(30) Priority: **02.06.89 SE 8902013**

(43) Date of publication of application:
**05.12.90 Bulletin  90/49**

(84) Designated Contracting States:
**BE CH DE DK ES FR GB IT LI NL**

(71) Applicant: **GAMBRO AB**
**Post Box 10101**
**S-220 10 Lund(SE)**

(72) Inventor: **Jönsson, Lennart**
**Ägovägen 7**
**S-240 20 Furulund(SE)**

(74) Representative: **Boberg, Nils Gunnar Erik**
**Gambro AB Patent Department Box 10101**
**S-220 10 Lund(SE)**

(54) **Suction device, preferably intended for autotransfusion.**

(57) Suction device, preferably intended for auto transfusion and including means for supplying diluent and/or anticoagulant for mixing with drawn up blood within the suction device.

The suction device is characterized by an integral valve arrangement with the help of which the mixing ratio can be varied.

Fig.1

Fig. 2

EP 0 400 463 A1

## SUCTION DEVICE, PREFERABLY INTENDED FOR AUTO TRANSFUSION

### TECHNICAL FIELD

The present invention relates to a suction device preferably intended for auto transfusion and including means for supplying diluent and/or anticoagulant for mixing of drawn up blood within the suction device. The suction device according to the invention is intended primarily to be used in an auto transfusion system corresponding particularly to that described in E-A2-223 126. It is however clear for the skilled man that it can be used in other contacts, for example in connection with open heart surgery in which the drawn up blood must first pass through an oxygenator before being returned to the patient.

### BACKGROUND ART

In the above mentioned E-A2-223 126 and in US-A-3 965 896 various auto transfusion systems are described which use a suction device which includes means for supplying diluents and/or anticoagulant for mixing with drawn up blood within the suction device. However, neither describes means for varying the mixing ratio between the supply liquid and the drawn up blood. It has now been shown in practice, however, that this mixing ratio is very important both for any after- treatment of the blood and for the final quality of the blood returned to the patient.

### DISCLOSURE OF THE INVENTION

The above mentioned problem is solved according to the invention by means of a valve arrangement within the suction device with the help of which the mixing ratio can be varied by the operator.

Preferably, the valve is so constructed that said liquid alternatively can be directed to the same suction sites for rinsing of the latter.

According to a practical embodiment of the suction device according to the invention, this includes a suction device which at one end terminates with a suction tip and at the other end is incased in a handpiece with a longitudinal conduit having at least three connection openings, namely one for connection of the suction pipe, one for connection to a source of said liquid and one for connection to a collection point for the drawn up mixture of blood and liquid. This practical embodiment is particularly characterized in that the suction pipe is slideably arranged in the longitudinal conduit of the hand piece in such a way that it can effect the complete or at least partial covering of the connection opening to the said liquid source for regulating the quantity of supplied liquid.

Particular advantages are hereby attained if the suction pipe is arranged to be able to cover the connection opening to said collection point, whilst, at the same time, uncovering the connection opening to said liquid source for rinsing the suction site by the preferably permanently open connection opening to the suction pipe.

Said longitudinal conduit is suitable closed off at one end by being in close contact with the suction pipe via a sliding fit or a sliding seal, whilst the other end can be closed off with a seal, against which the suction pipe can be brought to a sealing position. Said connection openings to the liquid source and to the collection point, respectively, can hereby be arranged in the longitudinal conduit wall in such positions that they can be passed by the suction pipes open inner end or one or some other openings in the same when the suction pipe is slid within the longitudinal conduit.

The sliding of the suction pipe is suitably achieved against the action of a manually adjustable spring which is ar ranged to hold the suction pipe in a particular position in relation to the hand piece, but which at adjustment slides the suction pipe for uncovering, respectively closing, of desired connection openings.

A very simple, and at the same time, easily manageable construction is obtained if said spring is formed as a hoop whose one end is attached to the suction pipe and whose other end is attached to a substantial tubular handpiece section, whereby the spring can be said to form an essential part of the hand piece.

### BRIEF DESCRIPTION OF DRAWINGS

A useful embodiment of the suction device according to the invention is described in the following with reference to the attached drawings which further show some detail variations.

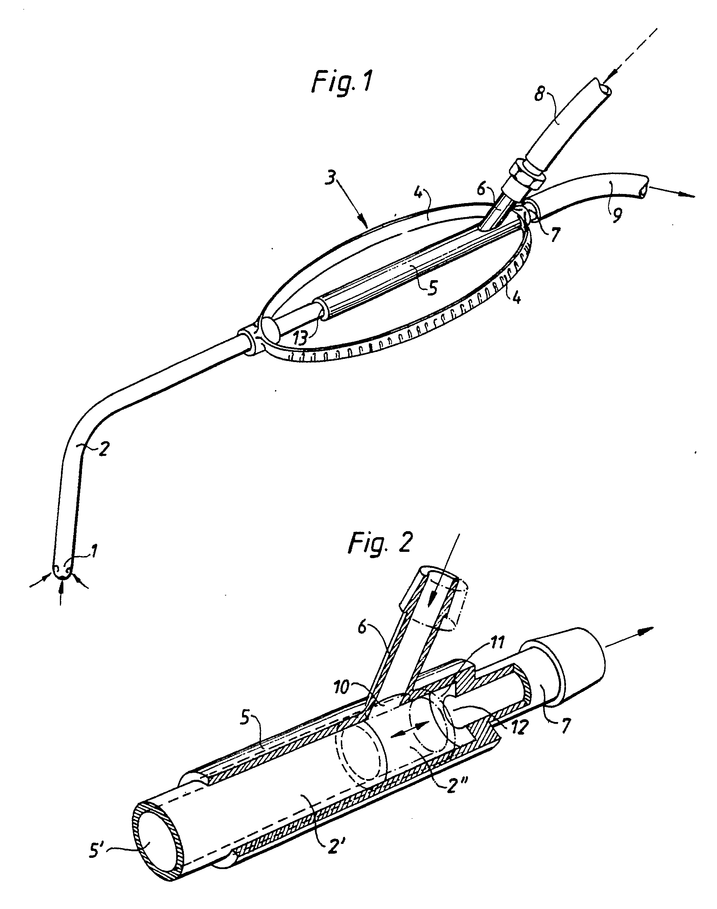

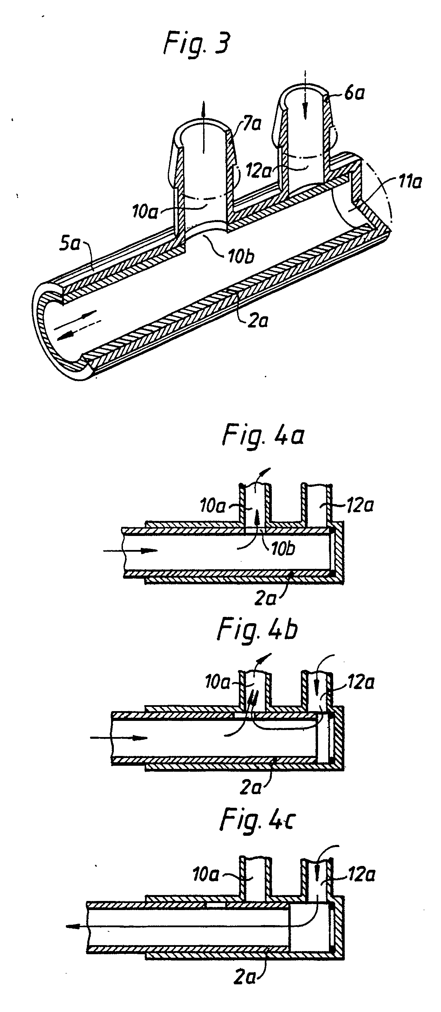

Fig. 1 hereby shows a complete suction device, whilst the figures 2 and 3 show two alternative inlet valve constructions of the suction device. Finally Figs. 4a-4c schematically show one of these constructions in three various positions.

### PREFERRED MODES OF CARRYING OUT THE INVENTION

The suction device shown in Fig. 1 includes a suction tip 1 formed at a remote end of the suction pipe 2 which is slideably arranged in the handpiece, which is in its entirety denoted by 3. This hand piece consists of two outer hoop-shaped parts 4 and an inner tubular handpiece section 5. The latter shown in more detail in Fig. 2 and accordingly has the shape of a cylinder 5 with two connection nipples 6 and 7 respectively. The nipple 6 is in connection via a hose 8 to a source (not shown) of said diluent and/or anticoagulant. In the same manner the nipple 7 is connected via a hose 9 to a collection point (not shown) for the drawn up mixture of blood and liquid.

The suction pipe 2 is shown in Fig. 2 by dashed lines in two different positions, denoted by 2' and 2" respectively. In position 2' a vacuum source arranged in connection with said collection point can achieve a suction through the hose 9 for simultaneously draw up blood through the suction pipe 2 and said liquid through the hose 8. Accordingly, a maximum dilution is achieved. Should a limited dilution be desired, the suction pipe 2 can be partially slid towards the position 2" so that it more or less covers the inner opening 10 in the nipple 6. Should the suction pipe completely cover this opening, then the dilution, of course, is totally stopped. In addition to both the connection openings 10 and 12 the handpiece section 5 also includes a third connection of opening 13, which is shown in Fig. 1, that is, the opening in which the suction pipe 2 is introduced.

An alternative embodiment of the handpiece section is shown in Fig. 3 and is denoted by 5a. This is provided with two somewhat different located nipples 6a and 7a corresponding to the nipples 6 and 7, that is, for connection to said liquid source and the collection point respectively. The suction pipe 2a is shown in Fig. 3 in its furthest ended position in sealing contact with an inner sealing surface 11a. Owing to the suction pipe 2a being provided with a connection opening 10b just in front of the inner opening 10a of the nipple 7a, a drawing up of blood without dilution can occur in this position. The same position is further shown schematically in Fig. 4a. On the other hand, in Fig. 4b, the suction pipe 2a has been slid somewhat to the left so that the opening 12a is partially uncovered whilst, at the same time the opening 10a is partially covered. In this position a dilution of the drawn up blood is achieved. Finally, in Fig. 4c suction pipe 2a has been slid further to the left so that the opening 12a is totally uncovered and the opening 10a is totally covered. In this position a rinsing of the suction site can take place with help of the liquid supplied to the opening 12a. If the liquid source is placed in a raised position, then the supply of liquid can be achieved solely by gravity.

Alternatively, the desired pressure can, of course, be achieved with help of a suitable pump.

Naturally, the invention is not limited simply to the embodiments described above but may be varied within the scope of the following claims. By way of example, if a more sensitive regulation is desired, then the employed connection openings can be extended in the sliding direction of the suction pipe.

## Claims

1. Suction device preferably intended for auto transfusion and including means for supplying diluent and/or anticoagulant for mixing with drawn up blood within the suction device, CHARACTERIZED BY a valve arrangement within the suction device (2, 4, 5) with help of which the mixing ratio can be varied.

2. Suction device according to claim 1, CHARACTERIZED IN that the valve is so constructed that the said liquid can alternatively be directed to the same suction site for rinsing of the latter.

3. Suction device according to claim 1 or 2 including a suction pipe (2), which at one end terminates with a suction tip (1) and which at the other end is incased in a handpiece (4, 5) with a longitudinal conduit (5') with at least three connection openings (10, 12, 13), namely one (13) for connection to the suction pipe (2), one (10) for connection to a source for said liquid and one ('2) for connection to a collection point for the drawn up mixture of blood and liquid, CHARACTERIZED IN that the suction pipe (2) is slideably arranged in the longitudinal canal of the handpiece (5') in such a way that it can effect the complete or at least partial covering of the connection opening (10) to said liquid source (5) for regulating the supplied liquid quantity.

4. Suction device according to claim 3, CHARACTERIZED IN that the suction pipe (2) is arranged to be able to cover the connection opening (12) to said collection point, whilst, at the same time, uncovering the connection opening (10) to said liquid source for rinsing the suction site via the permanently open connection opening (13) to the suction pipe (2).

5. Suction device according to claim 3 or 4, CHARACTERIZED IN that said longitudinal conduit (5') is closed off at one end by being in closed contact with the suction pipe (2a) via a sliding fit, whilst the other end can be closed off with a seal (11a), against which the suction pipe (2a) can be brought into sealing position, said connection openings (10a, 12a) respectively to the liquid source and to the collection point respectively being arranged in the conduits (5') longitudinal wall in such positions that they can be passed by the suction (2a) open inner end or one or some other openings in the same pipes when the suction pipe (2a) is slid within the longitudinal conduit (5′).

6. Suction device according to one of claims 1-6, CHARACTERIZED BY a manually adjustable spring (4, 4), which is arranged to hold the suction pipe (2) in a particular position in relation to the handpiece (3), but which by adjustments slides the suction pipe for uncovering, respectively closing, of desired connection openings.

7. Suction device according to claim 6, CHARACTERIZED IN that said spring is formed as a hoop whose one end is attached to the suction pipe (2) and whose other end is attached to a substantial tubular handpiece section (5), whereby the spring can be said to form an essential part of the handpiece (3).

*Fig. 1*

*Fig. 2*

*Fig. 3*

*Fig. 4a*

*Fig. 4b*

*Fig. 4c*

| | **DOCUMENTS CONSIDERED TO BE RELEVANT** | | EP 90109749.3 |
|---|---|---|---|

| Category | Citation of document with indication, where appropriate, of relevant passages | Relevant to claim | CLASSIFICATION OF THE APPLICATION (Int Cl⁵) |
|---|---|---|---|
| X | <u>DE - A1 - 3 309 916</u><br>(SMITHS INDUSTRIES P.L.C.)<br>   * Page 10, lines 22ff; fig.<br>    5a-c * | 1,2 | A 61 M 1/00 |
| A |    * Page 10, lines 22ff; fig.<br>    5a-c *<br>-- | 4 | |
| Y | <u>GB - A - 1 586 089</u><br>(NATIONAL RESEARCH DEVELOP-<br>MENT CORPORATION)<br>   * Page 1, lines 70ff; fig.<br>    1 *<br>-- | 3,5 | |
| Y | <u>DE - A1 - 2 542 509</u><br>(AB BONNER HOSPITAL INDUS-<br>TRIES)<br>   * Page 6, lines 13ff; fig.<br>    1,2 *<br>-- | 3,5 | |
| A | <u>US - A - 3 802 662</u><br>(VIGUIER)<br>   * Especially fig. 1 *<br>---- | | TECHNICAL FIELDS SEARCHED (Int Cl⁵)<br><br>A 61 M 1/00<br>A 61 M 3/00 |

The present search report has been drawn up for all claims

| Place of search | Date of completion of the search | Examiner |
|---|---|---|
| VIENNA | 23-07-1990 | SCHNEEMANN |